Europäisches Patentamt

(19) European Patent Office

Office européen des brevets

(11) **EP 0 347 345 B2**

(12) **NOUVEAU FASCICULE DE BREVET EUROPEEN**

(45) Date de publication et mention de la décision concernant l'opposition:
**09.06.1999 Bulletin 1999/23**

(45) Mention de la délivrance du brevet:
**14.09.1994 Bulletin 1994/37**

(21) Numéro de dépôt: **89420209.2**

(22) Date de dépôt: **13.06.1989**

(51) Int. Cl.$^6$: **A61B 5/02**, A61M 1/16

(54) **Appareillage pour la prévision des effets secondaires chez un dialysé**

Vorrichtung zur Vorhersage von Nebeneffekten während einer Dialyse

Equipment for the prediction of secondary effects during dialysis

(84) Etats contractants désignés:
**BE CH DE ES FR GB IT LI NL SE**

(30) Priorité: **15.06.1988 IT 6756488**

(43) Date de publication de la demande:
**20.12.1989 Bulletin 1989/51**

(73) Titulaire: **HOSPAL AG**
**4008 Basel (CH)**

(72) Inventeur: **Rossi, Marco**
**I-41036 Medolla (IT)**

(74) Mandataire: **Lejeune, Daniel**
**Hospal Service Brevets,**
**61, avenue Tony Garnier**
**69007 Lyon (FR)**

(56) Documents cités:
**FR-A- 2 368 094**     **US-A- 3 814 082**
**US-A- 4 610 257**     **US-A- 4 710 164**
**US-A- 4 718 891**

- **IEEE SPECTRUM. vol. 18, no. 4, 01 avril 1981, NEW YORK US page 45 - 49; A.F.Shackil: "Microprocessors and the M.D."**
- **ABC Technik und Naturwissenschaft, Verlag Harri Deutsch, Frankfurt/Main und Zürich, 1970, page 63**

**Description**

La présente invention est relative à un appareillage pour la prévision des effets secondaires qui se manifestent chez un patient soumis à un traitement de dialyse.

Plus précisément, la présente invention est relative à un appareillage qui permet de tenir sous contrôle un patient soumis à une dialyse, et ayant pour objet de prévoir l'apparition d'effets secondaires indésirables tels que, par exemple, la nausée, les vomissements, l'hypotension, la céphalée, et surtout le collapsus.

Dans l'état actuel, le problème d'éviter l'apparition des effets secondaires pendant la dialyse, est traité, mais non encore résolu d'une manière satisfaisante, en suivant l'une des trois modalités d'intervention fondamentales suivantes.

Selon une première modalité, le patient est soumis à une surveillance de la part d'un opérateur, généralement un infirmier, moyennant l'exécution de mesures périodiques (toutes les demi-heures, toutes les heures) de la tension artérielle, de la fréquence cardiaque et, dans certains cas, de la baisse pondérale.

Comme on peut l'observer, cette surveillance est discontinue et intermittente et la responsabilité de la surveillance est confiée exclusivement à l'opérateur qui intervient en fonction des constatations visuelles de l'état du patient ou même, après suggestion du patient lui-même. Il est évident que cette modalité ne permet pas des interventions correctives en temps opportun, propres à éviter chez le patient un malaise qui se présente souvent de manière soudaine.

Une deuxième modalité de contrôle exploite la connaissance du processus physiologique de la dialyse de manière à intervenir sur la structure des machines de dialyse pour leur permettre d'effectuer des traitements qui soient le moins perturbés possible. Les résultats sont nettement meilleurs que ceux obtenus selon la première modalité d'intervention citée ci-dessus.

Toutefois, il n'a pas encore été possible d'éviter l'apparition du collapsus (certainement le plus caractéristique et l'un des plus graves effets secondaires,) précisément parce que certaines machines sont programmées de manière à amener certains paramètres du traitement le plus près possible de ceux qui ont été retenus le plus adéquats (sur une base théorique) pour le patient soumis à une dialyse, sans exploiter les éventuels signaux provenant du patient lui-même. Même dans certains cas, les interventions éventuelles correctives et/ou préventives sont laissées à l'arbitraire de l'opérateur.

Une troisième modalité de contrôle prévoit de mettre en condition le traitement de dialyse en tenant compte des signaux particuliers constatés chez le patient. Une telle modalité fait l'objet de recherches dans le domaine de la dialyse et elle n'a pas encore donné lieu à des réalisations techniques concrètes satisfaisantes, soit à cause des difficultés pour identifier et pour mesurer certaines grandeurs physiques fondamentales en ce qui concerne l'état du patient, soit surtout à cause de la difficulté qu'il y a à définir une stratégie efficace d'élaboration et d'utilisation des signaux correspondants aux grandeurs citées ci-dessus. Un exemple de cette modalité est décrit dans le brevet US 4 710 164 selon lequel, les valeurs instantanées de la fréquence cardiaque et de la pression artérielle sont comparées à des valeurs-seuls en cas de dépassement des valeurs-seuils, les valeurs relatives au débit d'ultrafiltration et à la concentration en sodium du liquide de dialyse sont modifiées.

Cependant, la comparaison seule de valeurs instantanées de la fréquence cardiaque et de la pression artérielle avec des valeursseuils n'est pas suffisante pour prévenir l'apparition d'effets secondaires indésirables.

Le but de la présente invention est de proposer un appareillage capable de permettre la prévision, avec un bon niveau de fiabilité, de l'apparition des effets secondaires qui se manifestent chez un patient soumis à un traitement de dialyse, de façon à permettre à l'opérateur d'intervenir en temps utile, et/ou de mettre automatiquement en action une procédure qui empêche l'apparition de tels effets.

Ce but est atteint avec la présente invention qui est relative à un appareillage pour la prévision des effets secondaires qui se manifestent chez un patient pendant un traitement de dialyse.

Conformément à l'invention, un dispositif pour prévenir les effets secondaires qui peuvent se manifester chez un patient soumis à un traitement de dialyse comprend :

- des moyens pour mesurer au moins une grandeur hémodynamique du patient;
- des moyens de mémorisation pour stocker plusieurs valeurs successives de la grandeur hémodynamique ;
- des moyens de calcul pour calculer une pluralité de coefficients successifs, chaque coefficient étant représentatif de la variation moyenne, dans un intervalle de temps, de plusieurs valeurs successives de la grandeur hémodynamique stockées dans les moyens de mémoire;
- des moyens de comparaison pour comparer les coefficients calculés avec au moins une valeur de référence délimitant un domaine de valeurs de sécurité; et
- des moyens d'alarme pour émettre un signal d'alarme utilisable comme signal de commande chaque fois que le coefficient calculé sort du domaine de valeurs de sécurité.

Pour une meilleure compréhension de la présente invention, on décrit par la suite une forme de réalisation préférée, purement à titre d'exemple, non limitative et en référence aux dessins ci-joints, dans lesquels :

La figure n°1 est un schéma de blocs fonctionnels d'appareillage réalisé selon la présente invention ;

La figure n°2 illustre successivement trois signaux prélevés respectivement aux points a, b et c du schéma de la figure n°1, dans un cas clinique spécifique. En fonction du temps, la figure 2a montre quelle a été dans ce cas spécifique, l'évolution de la fréquence cardiaque après filtration ; la figure 2b, pour les pentes "ai" de segments de droite successifs, les valeurs correspondantes calculées par le bloc de normalisation, entre - 1 et + 1, de la fréquence cardiaque ; et la figure 2c, la pression artérielle moyenne.

La figure n°3 illustre des représentations graphiques qui donnent des exemples d'élaboration de signaux effectués par les blocs selon le schéma de la figure n°1.

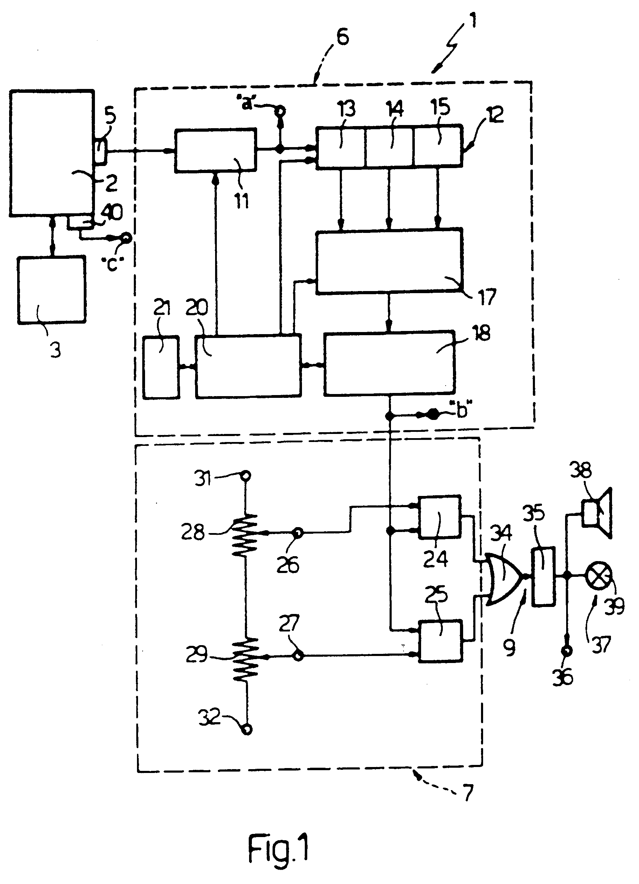

En se référant à la figure n°1, on indique dans son ensemble sous la référence 1, un appareillage pour la prévision des effets secondaires qui peuvent se manifester chez un patient 2 pendant un traitement de dialyse, effectué par l'intermédiaire d'une machine de dialyse 3 de type connu.

Selon la présente invention, l'appareillage 1 comprend essentiellement :

- un capteur 5 capable de mesurer une grandeur hémodynamique du patient, et de fournir un signal correspondant à la variation dans le temps de la grandeur hémodynamique elle-même ;
- une unité de traitement 6 du signal provenant du capteur 5 qui calcule au moins un indice correspondant à une variation du signal cité ci-avant, en se référant à une période de temps préétablie ;
- un circuit 7 qui compare la valeur obtenue pour l'indice cité ci-dessus avec au moins une valeur de référence délimitant un domaine préétabli de valeurs de sécurité ;
- et un circuit 9 qui émet un signal d'alarme utilisable en signal de commande chaque fois que la valeur atteinte par l'indice calculé par l'unité 6 dépasse le domaine des valeurs de sécurité délimité par la valeur de référence citée ci-dessus.

Au sens de la présente invention, on entend par "grandeur hémodynamique" une grandeur se rapportant aux conditions mécaniques de la circulation du sang, ainsi qu'une donnée pouvant être obtenue au moyen d'un électrocardiographe.

La description détaillée qui suit est effectuée en admettant que la grandeur hémodynamique mesurée est par exemple la fréquence cardiaque.

En tenant compte de ce qui a été dit plus haut, le capteur 5 peut être par exemple, constitué par un simple fréquencemètre, dont l'une des sorties est reliée à un filtre numérique passe-bas 11 faisant partie de l'unité d'élaboration 6.

Ce filtre a pour fonction essentielle d'éliminer les bruits pour ne laisser passer que les signaux physiologiques émis par le capteur 5, dans leur gamme de fréquence propre.

Cette unité d'élaboration 6 comprend un dispositif à mémoire glissante 12 d'une cellule à l'autre. Dans le cas décrit, il comprend par exemple trois cellules identiques 13, 14, 15, dont la première, 13, est reliée à la sortie du filtre 11.

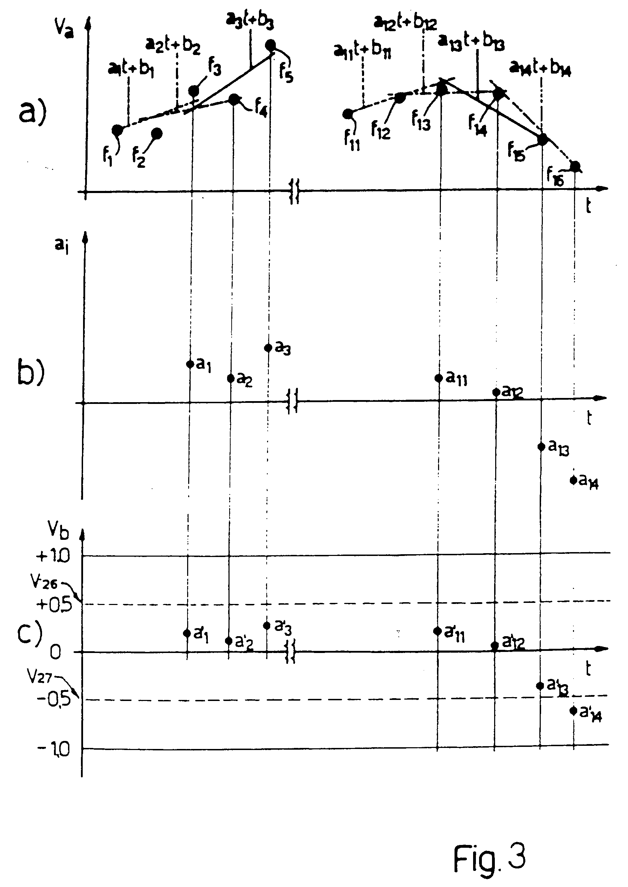

L'unité 6 comprend en outre un bloc de calcul 17 pourvu de trois entrées de signal reliées respectivement aux sorties parallèles des cellules 13, 14, 15, du dispositif à mémoire 12, et un bloc de normalisation 18 ayant une entrée reliée à la sortie du bloc 17 et une sortie reliée à une entrée du circuit de comparaison 7. Par exemple, le bloc de calcul 17 est en mesure d'effectuer une opération de régression linéaire aux minima carrés, dans le but de calculer les coefficients "ai" et "bi" ou pentes des segments de droite (voir la figure 3a), obtenus en considérant dans ce cas, trois valeurs de fréquence mesurées (celles qui sont mémorisées dans les cellules 13, 14, 15).

Le bloc de normalisation 18 a uniquement pour but de calculer, pour la pente "ai" de chaque segment de droite, une valeur correspondante "a'i" comprise entre - 1 et + 1 en appliquant, par exemple la formule suivante :

$$a'i = \frac{ai}{\sqrt{1 + ai^2}}$$

L'unité 6 enfin, est pourvue d'un bloc de gestion 20, comprenant par exemple, un micro-processeur qui est relié à une mémoire 21 et à tous les composants cités ci-dessus (titre 11, dispositif à mémoire 12, bloc de calcul 17 et bloc de normalisation 18) dans un but de coordonner la transmission des signaux entre les composants. L'unité 6 peut être avantageusement constituée par un micro-processeur.

Le circuit de comparaison 7 est de type traditionnel, puisqu'il est constitué d'une paire de comparateurs de seuil 24, 25, chacun d'eux ayant une première entrée reliée à la sortie de l'unité 6 (dans ce cas spécifique, le bloc de normalisation 18), et une deuxième entrée reliée respectivement par un curseur 26, 27 à un potentiomètre 28, 29.

Les potentiomètres 28, 29, sont reliés en série et ont des terminaux respectifs opposés reliés aux bornes 31, 32, auxquelles parviennent (de manière non illustrée) respectivement un pôle positif et un pôle négatif d'une source d'alimentation de type continu.

Les comparateurs de seuil 24, 25 sont prévus de manière que le signal présent aux sorties respectives est au niveau "0" logique, tant que le signal provenant de l'unité 6 est compris entre les valeurs de tension de référence prélevées par les curseurs 26, 27, tandis qu'il commute au niveau "1" logique, chaque fois que le signal provenant de l'unité 6 est respectivement supérieur ou inférieur aux valeurs correspondantes de référence citées ci-dessus.

Le circuit 9 qui émet un signal d'alarme, utilisable en signal de commande, est simplement constitué d'une porte logique 34 de type OR à deux entrées, respectivement reliées aux sorties des comparateurs 24 et 25, et d'une sortie reliée à un bloc amplificateur 35.

La sortie du bloc amplificateur 35 est reliée à une borne 36 ainsi qu'à des moyens d'alarme 37 constitués habituellement d'un avertisseur sonore 38 et d'une lampe 39.

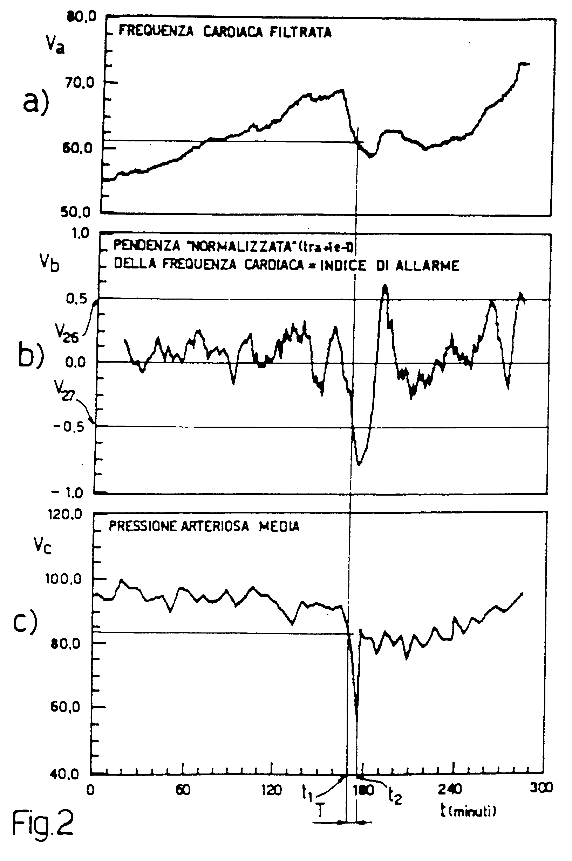

La sortie du filtre 11 passe-bas parvient aussi à une borne "a", ainsi que la sortie du bloc de normalisation parvient à une borne "b". En outre, on relie un patient 2 à un capteur 40 de tension artérielle, dont une sortie arrive à une borne "c". Dans les figures 2a, 2b, 2c, sont respectivement indiqués avec Va, Vb, Vc, les mouvements des signaux électriques prélevés aux bornes "a", "b", "c", citées ci-dessus, en fonction du temps exprimé en minutes.

Dans la figure 2b, V26 et V27 sont les deux valeurs de tension de seuil correspondantes prélevées en correspondance respectivement par les curseurs 26, 27 des potentiomètres 28, 29.

Dans la figure 3a sont illustrés, de façon schématique, deux exemples d'élaboration mathématique du signal Va (figure 3a), de manière à obtenir au début une pluralité de coefficients ai (figure 3b) correspondant aux inclinaisons des segments de droites obtenus par régression linéaire selon les modalités spécifiées ci-dessus, et, successivement, une normalisation entre -1 et +1 des valeurs obtenues à partir de ces coefficients selon l'illustration de la figure 3b. En fin de compte, l'appareillage selon la présente invention permet l'exécution, dans ce cas particulier, des opérations successives suivantes :

- mesurer la fréquence cardiaque du patient 2;
- élaborer un signal de fréquence mesuré de façon à obtenir au moins un indice corrélatif à la variation d'une telle fréquence en référence à une période de temps fixée préalablement;
- comparer la valeur obtenue à l'indice cité ci-avant avec au moins une valeur de référence délimitant un domaine de valeurs de sécurité ; et
- émettre un signal d'alarme utilisable en signal de commande chaque fois que la valeur obtenue de l'indice calculé s'écarte hors des limites du domaine de valeurs de sécurité délimité par la valeur de référence.

Plus en détail, le signal proportionnel à la fréquence cardiaque est relevé par l'intermédiaire du capteur 5 et est filtré dans le filtre 11 passe-bas, puis mémorisé, par groupe de trois valeurs chaque fois, dans le dispositif à mémoire gradué 12.

Par exemple, les trois premières valeurs de fréquence mesurées f1, f2, f3 (voir la figure 3a) sont mémorisées respectivement dans les cellules 15, 14, 13 du dispositif à mémoire 12 ; la valeur suivante f4 occupera la cellule 13, en déterminant le déplacement des valeurs f3 et f2 dans les cellules 15 et 14 et l'exclusion de la valeur f1, et ainsi de suite.

Pour chacun des groupes de trois valeurs de fréquence mémorisées dans le dispositif à mémoire 12 le bloc de calcul 17 prévoie la réalisation d'une régression linéaire selon ce qui a été spécifié, de façon à déterminer les coefficients "ai" et "bi" de la droite correspondante : par exemple, "a1" et "b1" pour la droite obtenue en tenant compte de f1, f2, f3, "a2 et "b2" pour la droite obtenue en tenant compte de f2, f3, f4 et ainsi de suite.

En procédant ainsi, on obtient une séquence de valeurs "ai" (voir la figure 3b) qui sont relevées à la sortie du bloc de calcul 17 et qui représentent, dans l'ensemble, la pente du signal de fréquence cardiaque prélevé au moyen du capteur 5 et filtré par le filtre 11.

L'opération suivante, qui a lieu dans le bloc de normalisation 18, consiste simplement en une régulation des valeurs "ai" relevées de manière à obtenir des valeurs correspondantes "a'i", dont l'amplitude est comprise entre -1 et +1, comme spécifié ci-dessus. L'étape suivante consiste en une vérification qui permet d'établir si chacune des valeurs "a'i" est comprise à l'intérieur d'un domaine de valeurs de sécurité, délimité supérieurement et inférieurement par les deux valeurs respectives de seuil V26 et V27. Cette opération est effectuée par le circuit de comparaison 7 à l'aide des comparateurs 24 et 25 et des potentiomètres 28 et 29.

Dans le cas où une des valeurs "a'i" sort des limites établies (voir par exemple la valeur "a'14" de la figure 3c), le comparateur 24 ou 25 envoie un signal de niveau logique "1" à l'entrée correspondante de la porte OR 34, en déterminant la commutation du signal respectif de sortie de "0" à "1" logique et l'émission consécutive d'un signal de commande de la part du bloc amplificateur 35.

Ce dernier déclenchera le fonctionnement d'un avertisseur sonore 38 et de la lampe 39, ainsi que l'envoi d'un signal électrique à la borne 36 utilisable pour des emplois ultérieurs, par exemple, pour modifier automatiquement certains paramètres de la dialyse effectuée par la machine 3.

La figure no 2 est relative à un cas réel, dans lequel la grandeur hémodynamique "fréquence cardiaque" est mesurée tous les 10 battements et est filtrée avec un filtre numérique passe-bande de type conventionnel, et l'indice d'alarme, constitué par "l'inclinaison normalisée", est obtenu au moyen d'une régression linéaire sur 15 échantillons (dans ce cas, le registre à écoulement 12 devrait être constitué de 15 cellules).

En observant dans le détail cette figure, on remarque que la sortie du signal Vb au-delà du seuil inférieur de sécurité V27 se produit en un instant t1, qui précède d'un intervalle de temps T (de l'ordre de 7 minutes environ), l'instant suivant t2 en correspondance duquel la tension artérielle moyenne du patient subit une baisse soudaine, laquelle provoque chez le patient un collapsus cardio-circulatoire.

En examinant un cas réel, comme celui qui est reporté dans la figure 2, on remarque que ressort nettement l'importance de l'action de prévision obtenue avec l'appareillage réalisé selon la présente invention.

En effet, la communication d'une anomalie est fournie dès l'instant t1, alors que les valeurs effectives de fréquence cardiaque (environ 61 battements/minute) et la tension artérielle moyenne (environ 83 mm Hg) peuvent être encore considérées pleinement dans la norme pour le type de patient soumis à une dialyse. Ceci démontre que l'analyse de la donnée instantanée fournie par des instruments en état de mesurer ces grandeurs s'avère insuffisante pour le but que se propose d'atteindre la présente invention.

De l'examen des caractéristiques de l'appareillage réalisé selon la présente invention, il en ressort des avantages évidents :

Avant tout on obtient une information utile et surtout suffisamment anticipée de l'apparition d'une situation physiologique critique. Ceci permet d'appliquer à temps des contre-mesures, de manière à éviter au patient un collapsus cardio-circulatoire, avec toutes les conséquences négatives qu'une telle situation comporterait (surtout chez les patients affectés de pathologie cardiaque).

L'appareillage fournit au technicien des données qui lui permettent de prévoir à temps une intervention automatique, opportune et efficace, par exemple sur la procédure de dialyse mise en oeuvre par la machine 3, en signalant à l'opérateur, simplement l'état d'urgence probable.

Il est clair enfin, qu'à l'appareillage 10 ci-dessus décrit, on peut apporter des modifications et des variantes sans dépasser le cadre de la présente invention.

Avant tout l'analyse du signal peut être beaucoup plus complexe si l'on créer par exemple un ensemble d'indices dont chacun d'eux doit être comparé avec au moins un seuil correspondant.

Plus simplement, le nombre des échantillons de régression, c'est à dire le nombre des cellules du dispositif à mémoire glissante, pourrait varier soit d'une séance à l'autre, soit, dynamiquement en fonction des caractéristiques du signal.

En outre, même en ayant choisi comme valeur de mesure la fréquence cardiaque, d'autres grandeurs hémodynamiques peuvent être mesurées, desquelles peuvent être retenus un ou plusieurs paramètres (amplitude, fréquence, etc...) mis en relation dans le temps avec cette grandeur hémodynamique. A titre d'exemple, on pourrait effectuer des mesures et des calculs sur le signal d'amplitude cardiaque et sur le signal électrocardiographique, en utilisant convenablement un mesureur d'amplitude cardiaque non invasif et un moniteur électrocardiographique de type traditionnel.

## Revendications

1. Dispositif pour prévenir les effets secondaires qui peuvent se manifester chez un patient soumis à un traitement de dialyse comprenant des moyens (5) pour mesurer au moins une grandeur hémodynamique du patient, caractérisé en ce qu'il comporte :

   - des moyens de mémorisation (12) pour stocker plusieurs valeurs successives de la grandeur hémodynamique ;
   - des moyens de calcul (17, 18) pour calculer une pluralité de coefficients successifs (ai, ai'), chaque coefficient étant représentatif de la variation moyenne dans un intervalle de temps, de plusieurs valeurs successives de la grandeur hémodynamique stockées dans les moyens de mémoire (12) ;
   - des moyens de comparaison (7) pour comparer les coefficients calculés (ai, ai') avec au moins une valeur de référence (V26, V27) délimitant un domaine de valeurs de sécurité ; et
   - des moyens d'alarme (9) pour émettre un signal d'alarme utilisable comme signal de commande chaque fois que le coefficient calculé sort du domaine de valeurs de sécurité.

2. Dispositif selon la revendication 1, caractérisé en ce qu'il comporte des moyens (11) de filtration de signal disposés en aval des moyens de mesure (5).

3. Dispositif selon une des revendications 1 ou 2, caractérisé en ce que les moyens de mesure (5) comprennent au moins un dispositif de mesure de la fréquence cardiaque ou un dispositif d'électrocardiographie.

4. Dispositif selon une des revendications 1 à 3, caractérisé en ce qu'il comporte un microprocesseur (6) incluant les moyens de calcul (17, 18).

5. Dispositif selon une des revendications 1 à 4, caractérisé en ce que les moyens de comparaison (7) comportent au moins un comparateur de seuil (24, 25).

6. Dispositif selon la revendication 5, caractérisé en ce que les moyens de comparaison (7) comprennent au moins un élément (26, 27) de régulation d'une valeur de seuil de référence du comparateur de seuil (24, 25).

7. Dispositif selon une des revendications 1 à 6, caractérisé par le fait de comprendre des moyens de signalisation (37) d'anomalie actionnés par les moyens (9) d'émission de signal d'alarme.

8.  Dispositif selon la revendication 7, caractérisé en ce que les moyens de signalisation (37) sont de type acoustique (38) et/ou de type optique (39).

9.  Dispositif selon une des revendications 1 à 8, caractérisé en ce qu'il comprend des moyens de commande d'une machine de dialyse propre à être reliée, en fonctionnement, à un patient, ces moyens de commande étant pilotés par les moyens d'émission de signal d'alarme.

**Claims**

1.  Device for anticipating side effects which may manifest themselves in a patient undergoing a dialysis treatment, comprising means (5) for measuring at least one haemodynamic parameter of the patient, characterized in that it includes:

    -   memory means (12) for storing several successive values of the haemodynamic parameter;
    -   calculating means (17, 18) for calculating a plurality of successive coefficients (ai, ai'), each coefficient being representative of the mean variation, within a time interval, in several successive values of the haemodynamic parameter which are stored in the memory means (12);
    -   comparison means (7) for comparing the calculated coefficients (ai, ai') with at least one reference value (V26, V27) delimiting a range of safety values; and
    -   alarm means (9) for emitting an alarm signal which can be used as a control signal each time the calculated coefficient departs from the range of safety values.

2.  Device according to Claim 1, characterized in that it comprises signal-filtering means (11) disposed downstream from the measurement means (5).

3.  Device according to either of Claims 1 and 2, characterized in that the measurement means (5) comprise at least one device for measuring the cardiac rate or an electrocardiograph device.

4.  Device according to one of Claims 1 to 3, characterized in that it comprises a microprocessor (6) including the calculating means (17, 18).

5.  Device according to one of Claims 1 to 4, characterized in that the comparison means (7) comprise at least one threshold comparator (24, 25).

6.  Device according to Claim 5, characterized in that the comparison means (7) comprise at least one element (26, 27) for adjusting a threshold reference value of the threshold comparator (24, 25).

7.  Device according to one of Claims 1 to 6, characterized in that it comprises means (37) for signalling an anomaly, actuated by the means (9) for emitting the alarm signal.

8.  Device according to Claim 7, characterized in that the signalling means (37) are of the acoustic type (38) and/or of the optical type (39).

9.  Device according to one of Claims 1 to 8, characterized in that it comprises means for controlling a dialysis machine capable of being connected in operation to a patient, these control means being governed by the means for emitting the alarm signal.

**Patentansprüche**

1.  Vorrichtung zur Vorhersage der Nebeneffekte, die bei einem einer Dialysebehandlung unterworfenen Patienten auftreten können, mit Einrichtungen (5) zur Messung wenigstens einer hämodynamischen Größe des Patienten, dadurch gekennzeichnet, daß sie folgendes umfaßt:

    -   Speichereinrichtungen (12) zur Speicherung mehrerer aufeinanderfolgender Werte der hämodynamischen Größe;
    -   Recheneinrichtungen (17, 18) zur Berechnung mehrerer aufeinanderfolgender Koeffizienten (ai, ai'), wobei jeder Koeffizient die mittlere Abweichung von mehreren in den Speichereinrichtungen (12) gespeicherten, aufeinanderfolgenden Werten der hämodynamischen Größe in einem Zeitraum darstellt;
    -   Vergleichseinrichtungen (7) zum Vergleich der berechneten Koeffizienten (ai, ai') mit wenigstens einem Vergleichswert (V26, V27), welcher einen Bereich von Sicherheitswerten begrenzt; und
    -   Alarmeinrichtungen (9) zum Abgeben eines Alarmsignals, das jedes Mal, wenn der berechnete Koeffizient den Bereich von Sicherheitswerten verläßt, als Steuersignal verwendbar ist.

2.  Vorrichtung nach Anspruch 1, dadurch gekennzeichnet, daß sie Signalfiltereinrichtungen (11) aufweist, die den Meßeinrichtungen (5) nachgeschaltet sind.

3.  Vorrichtung nach Anspruch 1 oder 2, dadurch gekennzeichnet, daß die Meßeinrichtungen (5) wenigstens eine Vorrichtung zur Messung der Herzfrequenz oder eine Elektrokardiographie-Einrichtung umfassen.

4. Vorrichtung nach einem der Ansprüche 1 bis 3, dadurch gekennzeichnet, daß sie einen Mikroprozessor (6) mit Recheneinrichtungen (17, 18) aufweist.

5. Vorrichtung nach einem der Ansprüche 1 bis 4, dadurch gekennzeichnet, daß die Vergleichseinrichtungen (7) wenigstens einen Schwellwert-Komparator (24, 25) umfassen.

6. Vorrichtung nach Anspruch 5, dadurch gekennzeichnet, daß die Vergleichseinrichtung (7) wenigstens ein Element (26, 27) zur Regulierung eines Vergleichs-Schwellwertes des Schwellwert-Komparators (24, 25) umfassen.

7. Vorrichtung nach einem der Ansprüche 1 bis 6, dadurch gekennzeichnet, daß sie Einrichtungen (37) zur Meldung einer Anomalie umfaßt, die durch die Alarmsignalabgabeeinrichtungen (9) betätigt werden.

8. Vorrichtung nach Anspruch 7, dadurch gekennzeichnet, daß die Meldeeinrichtungen (37) vom akustischen Typ (38) und/oder vom optischen Typ (39) sind.

9. Vorrichtung nach einem der Ansprüche 1 bis 8, dadurch gekennzeichnet, daß sie Steuereinrichtungen für eine Dialysemaschine umfaßt, welche im Betrieb mit einem Patienten verbunden werden können und die durch die Alarmsignalabgabeeinrichtungen gesteuert werden.

Fig.1

Fig.2

Fig. 3